# EUROPÄISCHE PATENTSCHRIFT

(11) **EP 1 286 623 B1**
(45) Veröffentlichungstag und Bekanntmachung des Hinweises auf die Patenterteilung: **03.12.2008**
(21) Anmeldenummer: 00930956.8
(22) Anmeldetag: 09.06.2000
(51) Int. Cl.: A61B 17/80, A61B 17/72

(54) **IMPLANTAT AUS KUNSTSTOFF FÜR DIE OSTEOSYNTHESE**
OSTEOSYNTHESIS IMPLANT FROM A PLASTIC MATERIAL
IMPLANT EN MATIERE PLASTIQUE POUR OSTEOSYNTHESE

(43) Veröffentlichungstag der Anmeldung: 05.03.2003
(73) Patentinhaber: Synthes GmbH, 4436 Oberdorf (CH)
(72) Erfinder: HEHLI, Markus, CH-7276 Davos Frauenkirch (CH); FREI, Reto, CH-7270 Davos Platz (CH); DUDA, Georg, D-12209 Berlin (DE)
(74) Vertreter: Lusuardi, Werther
(86) Internationale Anmeldenummer: PCT/CH2000/000315
(87) Internationale Veröffentlichungsnummer: WO 2001/093768

(56) Entgegenhaltungen:
- EP-A- 0 574 091
- DE-U- 29 913 390
- US-A- 5 380 328

## Beschreibung

Die Erfindung betrifft eine Fixationsvorrichtung mit einem Implantat gemäss dem Oberbegriff des Anspruchs 1. Eine derartige Vorrichtung ist aus der DE-U-299 13 390 bekannt.

Das Implantat kann als Knochenplatte oder als Marknagel ausgebildet sein. In seiner plattenförmigen Ausführung kann es als interner Fixateur zur Osteosynthese wirken, z.B. am proximalen Humerus oder anderen gelenknahen Bereichen am Röhrenknochen.

Aus der US 5,476,467 BENOIST ist eine fächerartig gewellte Führungsschablone bekannt, um Kirschnerdrähte zu führen. Die Nachteile dieser Anordnung bestehen darin, dass die Fixationselemente (Kirschnerdrähte) lediglich parallel zueinander durch die Führungsschablone geführt werden können. Durch die gewellte Struktur der Schablone liegt diese nicht direkt auf dem Knochen auf, so dass die Länge der einzuführenden Drähte unnötig verlängert wird.

Hier will die Erfindung Abhilfe schaffen. Der Erfindung liegt die Aufgabe zugrunde, ein osteosynthetisches Implantat zu schaffen, welches noch keine durchgehenden Bohrungen zur Aufnahme von in den Knochen zu verankernden longitudinalen Fixationselementen besitzt, sondern lediglich eine Anzahl von Vertiefungen in seiner Oberfläche aufweist, welche als Positionier- und Führungshilfe dienen, um die Fixationselemente unter divergierenden Winkeln und sich gegenseitig kreuzend zuerst durch das Implantat und dann in den Knochen einzuführen.

Das Implantat erlaubt es, Fixationselemente unter divergierenden Winkeln und sich gegenseitig kreuzend in den Knochen einzuführen, so dass primär ein Wandern der intramedullär oder in der Spongiosa verlaufenden Fixationselemente sowohl nach proximal als auch nach distal verhindert wird.

Die Erfindung löst die gestellte Aufgabe mit einer Fixationsvorrichtung mit einem Implantat, die mit den in Anspruch 1 definierten, Merkmalen bestimmt ist.

Die vorzugsweise konisch oder zylindrisch ausgebildeten Vertiefungen in der Oberseite des Implantats dienen als Zentrierungen für das Durchbohren der Führungsplatte z.B. mit Kirschnerdrähten. Die Kirschnerdrähte weisen an ihrem vorderen Teil eine Bohrerspitze auf, deren Länge vorzugsweise mindestens der Dicke des plattenförmigen Implantats, beziehungsweise dem Durchmesser des marknagelförmigen Implantats entspricht. Im weiteren weisen die Kirschnerdrähte ein Aussengewinde auf, das zur axialen Verschiebesicherung im Implantat dient, so dass ein Wandern der Kirschnerdrähte im Implantat verhindert wird. Die Kirschnerdrähte können in einem vom Chirurgen frei wählbaren Winkel zur Oberfläche des Implantats in dieses eingebohrt werden, vorzugsweise in zueinander windschiefen Richtungen, um eine optimale Frakturfixation zu erzielen. Lage und Winkel der Kirschnerdrähte können somit entsprechend der zu versorgenden Fraktur gewählt werden.
Damit ist der Vorteil erzielbar, dass eine minimal invasive Operationstechnik angewendet werden kann, mit minimal zu inserierendem Implantatmaterial. Das erfindungsgemässe Implantat eignet sich insbesondere - wegen der Möglichkeit die Fixationselemente in drei Dimensionen anzuordnen - zur Osteosynthese am osteoporotischen oder durch Krankheit geschwächten Knochen. Die Stabilität der Osteosynthese wird somit primär durch die Bolzen/Drähte und ihre kreuzweise Positionierung im Knochen erbracht. Durch das direkte Anliegen des Implantats am Knochen wird die freie Länge der einzuführenden Drähte auf ein Minimum reduziert. Dadurch wird eine frühe Belastung der Frakturstelle möglich und somit eine frühere Nutzung der betroffenen Gliedmassen und im Idealfall eine schnellere Heilung.

Eine bevorzugte Weiterbildung besteht darin, dass das Implantat aus einem bioresorbierbaren oder biodegradierbaren Kunststoff besteht, welches vorzugsweise aus der Gruppe der Polylaktate ausgewählt wird. Die Vertiefungen sind zweckmässigerweise in einem regelmässigen Raster auf der Oberseite des Implantats angeordnet.

Bei einer Ausgestaltung als Knochenplatte, bzw. als plattenförmiger Führungskörper weist das Implantat bevorzugt eine annähernd kreisförmige Oberseite auf, mit auf konzentrischen Kreisen angeordneten Vertiefungen. Die Anzahl der Vertiefungen liegt zwischen 3 und 100, vorzugsweise zwischen 7 und 40. Typischerweise sind 10 Vertiefungen vorgesehen. Zweckmässigerweise erweitern sich die Vertiefungen konisch gegen die Oberseite hin. Die konischen Vertiefungen weisen vorteilhafterweise einen Konuswinkel im Bereich von 30° bis 120°, vorzugsweise von 40° bis 100° auf. Die Vertiefungen weisen an der Oberseite einen Durchmesser im Bereich von 1,0 bis 3,0 mm, vorzugsweise zwischen 1,5 bis 2,2 mm auf (typischerweise 2 mm). Die Tiefe der Vertiefungen liegt im Bereich von 0,6 bis 1,5 mm, vorzugsweise zwischen 0,8 bis 1,2 mm (typischerweise 1 mm).

Bei der bevorzugten Ausführung als plattenförmiges Implantat ist dessen Oberseite vorzugsweise konvex ausgebildet, um eine bessere Anpassung an die Knochenoberfläche zu erzielen. Seine Dicke liegt zwischen 2 bis 6 mm, vorzugsweise zwischen 2,5 bis 4,0 mm. Die Oberseite weist zweckmässigerweise eine Fläche im Bereich von 3 bis 15 cm², vorzugsweise 4 bis 10 cm² auf (typischerweise 4,5 cm²).

Bei einer bevorzugten Ausführungsform weist das mit dem Implantat verwendete Fixationselement eine Bohrerspitze auf mit einer Länge von 4 bis 10 mm, vorzugsweise von 5 bis 8 mm. Die Bohrerspitze sollte vorzugsweise mindestens der Dicke des Implantats entsprechen.
Das Fixationselement ist zweckmässigerweise mit einem Aussengewinde versehen, vorzugsweise über eine Länge von 30 bis 80 mm. Es weist an seinem hinteren Ende vorzugsweise keinen Kopf auf und besitzt einen einheitlichen Durchmesser über die gesamte Länge gesehen, vorzugsweise im Bereich von 1 bis 6 mm (typischerweise im Bereich von 2 bis 5 mm).

Die Erfindung und Weiterbildungen der Erfindung werden im folgenden anhand der teilweise schematischen Darstellungen eines Ausführungsbeispiels noch näher erläutert.
Es zeigen:
Fig. 1 eine perspektivische Darstellung eines als Führungskörper ausgebildeten plattenförmigen Implantats;
Fig. 2 einen Querschnitt durch das Implantat längs der Linie II-II in Fig. 1 im Bereich zweier Vertiefungen;
Fig. 3 eine Ansicht eines Fixationselementes in Form eines Kirschnerdrahtes; und
Fig. 4 eine Ansicht eines frakturierten Humerus mit einem Implantat, das mit einer Anzahl darin eingebohrter Kirschnerdrähte am Knochen befestigt ist.

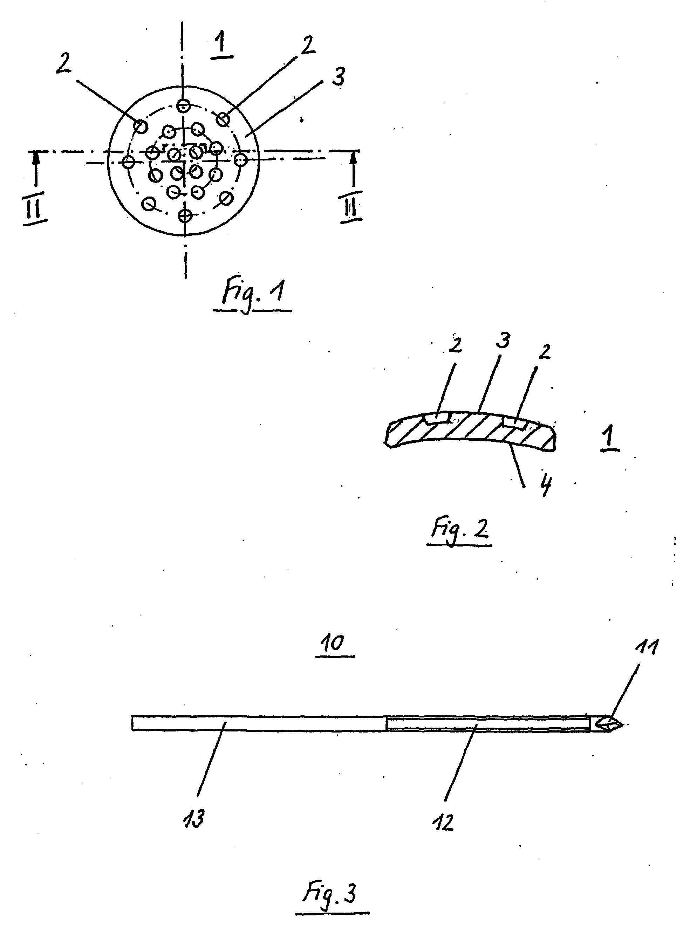

Das in den Fig. 1 und 2 dargestellte Implantat 1 besteht aus einer 3 mm dicken, gewölbten Platte aus einem für die Implantation im menschlichen Körper geeigneten biodegradierbaren Kunststoff, insbesondere einem Polylactat.
Das Implantat 1 weist eine konvexe Oberseite 3 sowie eine für den Knochenkontakt bestimmte konkave Unterseite 4 auf. In der Oberseite 3 sind eine Vielzahl von Vertiefungen 2 in Form von sich gegen die Oberseite 3 hin konisch erweiternden Trichtern eingelassen.
Die Unterseite 4 des Implantats 1 ist vorzugsweise der zu applizierenden Knochenoberfläche angepasst, um eine möglichst gute und weitflächige Kontaktfläche zum Knochen zu erreichen.

Das in Fig. 3 dargestellte Fixationselement 10 in Form eines herkömmlichen Kirschnerdrahtes weist an seinem hinteren Ende 13 keinen Kopf auf, so dass es einen einheitlichen Durchmesser über seine gesamte Länge aufweist. An seinem vorderen Ende weist das Fixationselement 10 eine Bohrerspitze 11 sowie ein Aussengewinde 12 auf.

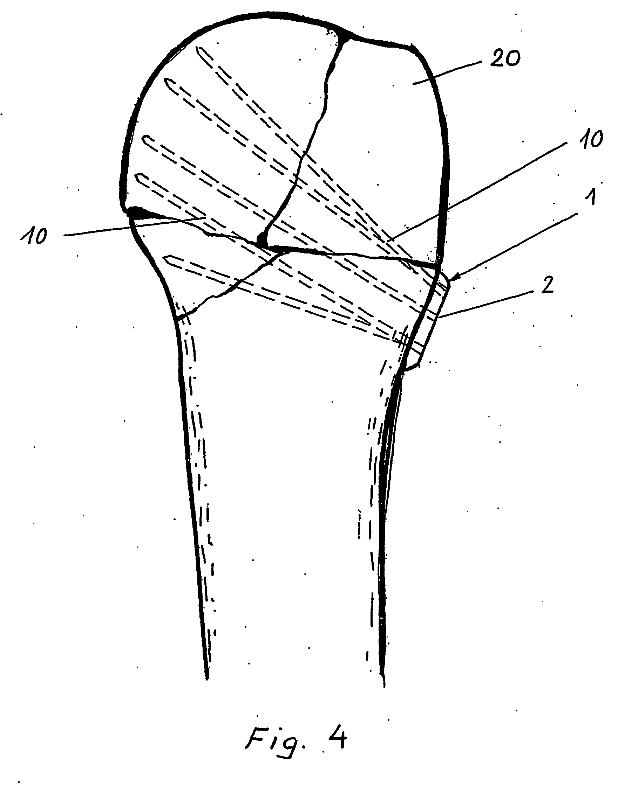

Das Implantat 1 lässt sich durch eine minimale Inzision in den Körper einführen, z.B. im Bereich des proximalen Humerus 20 (Fig. 4), an welchem es mit den Fixationsmitteln 10 befestigt werden kann. Die zusätzliche Verwendung von Knochenzement ist dabei nicht ausgeschlossen. Da das plattenförmige Implantat 1 über genügend Vertiefungen 2 verfügt, können diese dazu dienen, Bänder an Knochenfragmenten des Humerus 20 zu befestigen. Die dreidimensionale, windschiefe Anordnung der Fixationsmittel 10 verhindert dabei deren Lockerung, so dass insgesamt eine stark verbesserte Stabilität der Fixation resultiert.

## Patentansprüche

1. Fixationsvorrichtung für die Knochenchirurgie mit
A) einem plattenförmigen oder marknagelförmigen Implantat (1) aus Kunststoff, welches zur Aufnahme von in den Knochen zu verankernden, longitudinalen Fixationselementen (10), insbesondere in Form von Drähten, Nägeln, Stiften oder Schrauben dient, wobei die zur Aufnahme der Fixationselemente (10) bestimmten Öffnungen das Implantat (1) nicht vollständig durchdringen, sondern zu Vertiefungen (2) in der Oberfläche des Implantats (1) degeneriert sind, so dass sie als Positionier- und Führungs-Hilfe für die durch das Implantat (1) hindurchzuführenden Fixationselemente (10) dienen; sowie
B) mindestens einem Fixationselement (10) zur Befestigung des Implantats (1) an oder in einem Knochen,
**dadurch gekennzeichnet, dass**
C) das Fixationselement (10) eine Bohrerspitze (11) aufweist; und
D) die Länge der Bohrerspitze (11) mindestens der Dicke des plattenförmigen Implantats (1), beziehungsweise dem Durchmesser des marknagelförmigen Implantats (1) entspricht.

2. Fixationsvorrichtung nach Anspruch 1, **dadurch gekennzeichnet, dass** die Bohrerspitze (11) eine Länge von 4 bis 10 mm, vorzugsweise von 5 bis 8 mm aufweist.

3. Fixationsvorrichtung nach Anspruch 1 oder 2, **dadurch gekennzeichnet, dass** das Fixationselement (10), vorzugsweise über eine Länge von 30 bis 80 mm, mit einem Aussengewinde (12) versehen ist.

4. Fixationsvorrichtung nach einem der Ansprüche 1 bis 3, **dadurch gekennzeichnet, dass** das Fixationselement (10) an seinem hinteren Ende (13) keinen Kopf aufweist.

5. Fixationsvorrichtung nach einem der Ansprüche 1 bis 4, **dadurch gekennzeichnet, dass** das Fixationselement (10) einen einheitlichen Durchmesser über die gesamte Länge gesehen besitzt.

6. Fixationsvorrichtung nach einem der Ansprüche 1 bis 5, **dadurch gekennzeichnet, dass** das Fixationselement (10) einen Durchmesser im Bereich von 1 bis 6 mm, vorzugsweise von 2 bis 5 mm aufweist.

7. Fixationsvorrichtung nach einem der Ansprüche 1 bis 6, **dadurch gekennzeichnet, dass** das Implantat (1) die Aufnahme mehrerer Fixationselemente (10) unter divergierenden Winkeln, vorzugsweise in zueinander windschiefen Richtungen erlaubt.

## Claims

1. Fixation device for bone surgery, with
A) a plate-like or medullary nail-like implant (1) made of a plastic material, used to receive longitudinal fixation elements (10) to be anchored in the bone, especially in the form of wires, nails, rods or screws, where the openings destined to receive the fixation elements (10) are not fully penetrating the implant (1), but are degenerated to recesses (2) in the surface of the implant (1), so as to serve as positioning and guiding aids for the fixation elements (10) to be passed through the implant (1); as well as
B) at least one fixation element (10) to fasten the implant (1) at or in a bone,
**characterized in that**
C) the fixation element (10) presents a drill bit (11); and
D) the length of the drill bit (11) matches at least the thickness of the plate-like implant (1) or the diameter of the medullary nail-like implant (1).

2. Fixation device according to claim 1, **characterized in that** the drill bit (11) presents a length of 4 to 10 mm, preferably of 5 to 8 mm.

3. Fixation device according to claim 1 or 2, **characterized in that** the fixation element (10) is fitted, preferably over a length of 30 to 80 mm, with an external thread (12).

4. Fixation device according to one of the claims from 1 to 3, **characterized in that** the fixation element (10) does not has a head at its rear end (13).

5. Fixation device according to one of the claims from 1 to 4, **characterized in that** the fixation element (10) has a uniform diameter over its entire length.

6. Fixation device according to one of the claims from 1 to 5, **characterized in that** the fixation element (10) has a diameter in the range of 1 to 6 mm, preferably of 2 to 5 mm.

7. Fixation device according to one of the claims from 1 to 6, **characterized in that** the implant (1) allows receiving several fixation elements (10) under diverging angles, preferably in directions skewed toward each other.

## Revendications

1. Dispositif de fixation pour chirurgie osseuse comprenant
A) un implant (1) de matière plastique en forme de plaque ou de clou intramédullaire, lequel implant sert à loger des éléments de fixation longitudinaux (10) à ancrer dans les os, en particulier sous la forme de fils, de clous, de broches ou de vis, dans lequel les ouvertures destinées à loger les éléments de fixation (10) ne traversent pas complètement l'implant (1), mais sont restreintes à des évidements (2) dans la surface de l'implant (1), si bien qu'elles servent de dispositif de positionnement et de guidage pour les éléments de fixation (10) à faire passer à travers l'implant (1) ; et
B) au moins un élément de fixation (10) pour la fixation de l'implant (1) sur ou dans un os,
**caractérisé en ce que**
C) l'élément de fixation (10) présente une mèche (11) ; et
D) la longueur de la mèche (11) correspond au moins à l'épaisseur de l'implant en forme de plaque (1), ou bien au diamètre de l'implant en forme de clou intramédullaire (1).

2. Dispositif de fixation selon la revendication 1, **caractérisé en ce que** la mèche (11) présente une longueur de 4 à 10 mm, de préférence de 5 à 8 mm.

3. Dispositif de fixation selon la revendication 1 ou 2, **caractérisé en ce que** l'élément de fixation (10) est pourvu d'un filetage (12), de préférence sur une longueur de 30 à 80 mm.

4. Dispositif de fixation selon l'une quelconque des revendications 1 à 3, **caractérisé en ce que** l'élément de fixation (10) ne présente pas de tête au niveau de son extrémité arrière (13).

5. Dispositif de fixation selon l'une quelconque des revendications 1 à 4, **caractérisé en ce que** l'élément de fixation (10) possède un diamètre uniforme sur toute la longueur.

6. Dispositif de fixation selon l'une quelconque des revendications 1 à 5, **caractérisé en ce que** l'élément de fixation (10) présente un diamètre dans la gamme de 1 à 6 mm, de préférence de 2 à 5 mm.

7. Dispositif de fixation selon l'une quelconque des revendications 1 à 6, **caractérisé en ce que** l'implant (1) permet de loger plusieurs éléments de fixation (10) sous des angles divergents, de préférence selon des directions non coplanaires les unes par rapport aux autres.
